# EUROPÄISCHE PATENTSCHRIFT

(11) **EP 2 108 347 B1**
(45) Veröffentlichungstag und Bekanntmachung des Hinweises auf die Patenterteilung: **23.08.2017**
(21) Anmeldenummer: 08007250.7
(22) Anmeldetag: 11.04.2008
(51) Int. Cl.: A61F 9/008

(54) **System für die refraktive ophtalmologische Chirurgie**
System for refractive ophthalmologic surgery
Système pour la chirurgie ophtalmologique réfractive

(43) Veröffentlichungstag der Anmeldung: 14.10.2009
(73) Patentinhaber: WaveLight GmbH, 91058 Erlangen (DE)
(72) Erfinder: Vogler, Klaus, 90542 Eckental (DE)
(74) Vertreter: von Hellfeld, Axel

(56) Entgegenhaltungen:
- EP-A- 0 697 611
- EP-A- 1 231 496
- EP-A2- 1 034 756
- WO-A-2006/087180
- DE-A1- 10 323 422
- US-A1- 2007 073 905
- US-A1- 2007 282 313

## Beschreibung

Die Erfindung betrifft ein System für die refraktive ophthalmologische Chirurgie.

In der refraktiven ophthalmologischen Chirurgie werden die optischen Brechungseigenschaften des Auges, insbesondere der Kornea, durch Einsatz von Laserstrahlung geändert, um Fehlsichtigkeiten zu korrigieren oder zumindest zu lindern. Ein prominentes Beispiel der refraktiven ophthalmologischen Chirurgie ist die LASIK, bei der Korneagewebe abgetragen (ablatiert) wird, um die Kornea zur Korrektur von Sehfehlern neu zu formen. Zum Abtrag von Korneagewebe wird in der Regel ein Excimerlaser im UV-Bereich (typischerweise 193 nm) eingesetzt. Die Laserstrahlung wird bezüglich Zeit und Ort so über das Auge geführt, dass an ausgewählten Stellen der Kornea eine bestimmte Menge Gewebe abgetragen wird. Dieser Abtrag wird durch das sogenannte Ablationsprofil beschrieben, d.h. das Ablationsprofil gibt den an jeder Stelle der Kornea vorzunehmenden Abtrag (Ablation) an.

Das Ablationsprofil wird in der Regel vor Durchführung des chirurgischen Eingriffs für das zu korrigierende Auge berechnet. Grundlage dieser Berechnung ist eine Vermessung des Auges in seinem Ist-Zustand. Für diese Vermessung des Auges kennt der Stand der Technik unterschiedliche Techniken, insbesondere Topographiemessgeräte (sogenannte Topolyzer), Wellenfrontanalysatoren, Scheimpfluggeräte und auch Pachymeter.

Eine refraktive ophthalmologische Chirurgie mit einem Wellenfrontanalysator oder einem Topographiemessgerät ist kaum in der Lage, kleine lokale Strukturen der Hornhaut im Millimeterbereich gut aufzulösen, geschweige denn definiert und lokal exakt zuzuordnen, um eine ortsgetreue Behandlung zu ermöglichen. Mit Topolyzern ist es auch kaum möglich, sogenannte "central islands", also Vorsprünge auf der Hornhaut, die oft aus vorangegangenen, nicht ganz perfekten Operationen aus der Frühzeit der PRK stammen, im Millimeterbereich zu detektieren.

Zur Zeit wird versucht, während der Ablation Abweichungen vom gewünschten Ablationsprozess "on-line" zu verfolgen, und zwar insbesondere Abweichungen, die auf einer sogenannten "Zyklotorsion" oder auf einem sogenannten "Pupil Center Shift" beruhen.

Stand der Technik findet sich, z. B., in der:
- EP 1 231 496 A2, die eine optische Kohärenztomographieunterstützte chirurgische Vorrichtung offenbart;
- EP 0 697 611 A, EP 1 231 496 A2, die eine optische Kohärenztomographieunterstützte chirurgische Vorrichtung offenbart;
- US 2007/0282313 A1, die ein Verfahren und eine Vorrichtung zum Führen einer Laser-Hornhautoperation mit einer optischen Messung offenbart;
- US 2007/0073905 A1 die auf einem parametrischen Modell beruhende ablative, chirurgische Systeme und Verfahren offenbart;
- WO 2006/087180 A2, die ein Verfahren zur Erstellung eines Ablationsprogramms, ein Verfahren zur Ablation eines Körpers und Mittel zur Durchführung der Verfahren offenbart;
- DE 103 23 422 A1, die eine Vorrichtung und ein Verfahren zur Messung eines optischen Durchbruchs in einem Gewebe offenbart; und
- EP 1 034 756 A2, die ein interaktives, korrigierendes System zur Laser-Augenoperation offenbart.

Mit diesen zur Zeit bekannten Verfahren ist es aber in der Regel nicht möglich, lokale Hornhautirregularitäten präzise zu detektieren und bei einer solchen Detektion den Laserstrahl präzise lokal nur an dieser Stelle zur Wirkung zu bringen und dabei auch noch das Ablationsergebnis zu verfolgen.

Seit einiger Zeit steht als Messverfahren zur berührungslosen Vermessung biologischer Gewebe die sogenannte optische Kohärenztomographie zur Verfügung, vgl. z. B. Wolfgang Drexler, Journal of Biomedical Optics, 9(1), 42-74, 2004. Mit der optischen Kohärenztomographie, insbesondere unter Einsatz von Breitbandstrahlern, ist es möglich, sehr feine biologische Strukturen zu vermessen, insbesondere mit Auflösungen im Bereich von 1 µm und feiner.

Der Erfindung liegt die Aufgabe zugrunde, ein System der eingangs genannten Art bereitzustellen, welches verbesserte Operationsergebnisse ermöglicht.

Hierzu lehrt die Erfindung ein System für die refraktive ophthalmologische Chirurgie gemäß des angehängten unabhängigen Anspruchs.

Wir beschreiben ein System für die refraktive ophthalmologische Chirurgie mit
a) einem ersten Laser zum Abtrag von Korneagewebe,
b) Mitteln zum zeit- und ortsgesteuerten Führen der Strahlung des ersten Lasers auf das Auge,
c) einer Einrichtung für eine optische Kohärenztomographie mit einem zweiten Laser zur Durchführung von optischer Kohärenztomographie am Auge,
d) Mitteln zum zeit- und ortsgesteuerten Führen der Strahlung des zweiten Lasers auf das Auge, und mit
e) einem Rechner, der
   e1) bei dem Abtrag von Korneagewebe gemäß einem Programm den ersten Laser und die genannten Mittel zum zeit- und ortsgesteuerten Führen der Strahlung des ersten Lasers auf das Auge zur Erzielung einer Neuformung der Kornea steuert,
   e2) die Einrichtung für die optische Kohärenztomographie steuert und so programmiert ist, dass vor Beginn, während und nach Beendigung des Abtrags von Korneagewebe Messungen an der Kornea durchgeführt werden, und
   e3) der unter vorgebbaren Bedingungen den Programmablauf für den Abtrag von Korneagewebe im Anschluss an eine Messung mittels der optischen Kohärenztomographie in Abhängigkeit von dem Ergebnis der Messung steuert.

Ausführungsformen der Erfindung integrieren also in ein System für die refraktive ophthalmologische Chirurgie ein Modul für die optische Kohärenztomographie (OCT) derart, dass quasi "on-line" die damit gewonnenen Messergebnisse in den Prozess des chirurgischen Eingriffs einfließen. Insbesondere die hoch auflösende und sehr schnelle ("high-speed") OCT (HHS-OCT) ermöglicht die Ermittlung und Darstellung der Kornea-Strukturen mit Auflösungen im µm-Bereich mit sehr hoher Geschwindigkeit durch Abtastraten im Bereich von einigen MHz bis zu mehreren GHz, insbesondere bis zu 10 GHz und sogar bis zu 100 GHz und mit Messzeiten deutlich kleiner als eine Sekunde. Damit ist es möglich, nicht nur vor dem Abtrag von Korneagewebe die Ausgangsstruktur und nach dem Abtrag die Endstruktur der Kornea zu vermessen, sondern auch den gesamten Behandlungsfortschritt dazwischen zu verfolgen und die Behandlung in Abhängigkeit von OCT-Messergebnissen, die während der Behandlung gewonnen werden, zu steuern.

Aufgrund der Integration des OCT-Moduls in das System für die refraktive Chirurgie ist es weiterhin möglich, bestimmte Irregularitäten der Hornhaut, insbesondere die sogenannten "central islands", zu erkennen und bei der Behandlung zu berücksichtigen. Solche "central islands", also irreguläre Vorsprünge auf der Hornhautoberfläche, haben Abmessungen im Bereich von wenigen Millimetern und kleiner und waren deshalb mit herkömmlichen Messmethoden bei der refraktiven Chirurgie kaum oder nicht erfassbar. Auch waren solche Irregularitäten nicht oder kaum vor oder während der Behandlung auffindbar und genau für die Lasersteuerung adressierbar. Entsprechendes gilt für ebenfalls bisweilen an der Hornhautoberfläche auftretende feinste Irregularitäten in Form von Narben.

Mit der breitbandigen optischen Kohärenztomographie sind solche Irregularitäten erkennbar und entsprechend kann das Ablationsprofil so gestaltet werden, dass zum Beispiel im Bereich von "central islands" (also Vorsprüngen auf der Hornhaut) gezielt lokal mehr Korneagewebe abgetragen wird als in anderen Bereichen der Kornea, sodass insgesamt eine glatte Oberfläche entsteht, während im Falle der genannten Narben der Abtrag im Bereich dieser Narben so stark reduziert wird, dass im Ergebnis ebenfalls eine im Wesentlichen glatte Korneafläche entsteht. Wenn im Stand der Technik solche Irregularitäten nicht im Messergebnis erkannt und bei der Ablation berücksichtigt worden sind, blieben sie im Verlauf der Ablation im Wesentlichen erhalten und führten zu entsprechenden Irregularitäten auch an oder in der behandelten Kornea.

Mit der erfindungsgemäßen Integration der OCT in das System für die refraktive ophthalmologische Chirurgie ist es möglich, die genaue Hornhautstruktur hinsichtlich Querschnitt, Dicke, Vorderfläche und Rückfläche zu detektieren und momentan (instantan) während der Behandlung zu verfolgen. Deshalb lehrt die gegenwärtige Offenbarung, während des Ablationsprozesses (also während der refraktiven Chirurgie) quasi in Echtzeit ("on-line") die jeweiligen momentanen Abbildungseigenschaften der Hornhaut, wie sie gerade momentan aufgrund der erreichten Ablationsstufe vorliegen, zu berechnen und die Ablation genau dann zu beenden, wenn die so "on-line" errechneten Abbildungseigenschaften des Auges einem gewünschten, vorgebbaren Ziel entsprechen. Das macht parallele Topographiemessungen oder auch Wellenfrontmessungen nicht mehr unbedingt erforderlich.

Das erfindungsgemäße System für die refraktive ophthalmologische Chirurgie sieht vor, dass der Rechner programmiert ist, um bei der optischen Kohärenztomographie gewonnene Messergebnisse vor und/oder während des Abtrags von Korneagewebe auf einer Anzeigeeinrichtung darzustellen. Auf diese Weise kann der behandelnde Arzt quasi "on-line", also praktisch in Echtzeit, den Fortschritt der Ablation graphisch dargestellt betrachten. Zum Beispiel kann dem Arzt auf dem Bildschirm mit einer Linie (bei zwei-dimensionaler Darstellung) oder mit einer Fläche (bei dreidimensionaler Darstellung) die Ausgangsform der Kornea (vor Beginn des Eingriffs) dargestellt werden und darunter dann entsprechend mit einer zum Beispiel farbig abgesetzten Linie bzw. Fläche sukzessive während des Eingriffs die momentane Korneastruktur. Dabei kann optional dem Arzt noch zusätzlich auch die augeninnenseitige Oberfläche der Kornea, die auch mit der Kohärenztomographie vermessbar ist, angezeigt werden. Dies hilft insbesondere, zu dünne Reststärken der Kornea zu vermeiden. Auch können weitere interessierende Strukturen des behandelten Auges, wie die Linse und/oder die Pupille, mit der OCT vermessen und auf der Anzeigeeinrichtung dargestellt werden.

Wird das vorstehend genannte Verfahren bei der LASIK eingesetzt und wird dabei das sogenannte "Flap"-Schnitt zum Beispiel mit einem Femtosekundenlaser durchgeführt, dann kann der entstehende Schnitt mit der OCT verfolgt und auf der Anzeigeeinrichtung dargestellt werden und es können im Verlauf der sich anschließenden Ablation von Korneagewebe die vorstehend genannten Linien bzw. Flächen in der graphischen Darstellung rechnerisch so ermittelt werden, dass die Situation nach Rückklappung des Flaps und dem angenommenen Heilvorgang berechnet und dargestellt werden.

Die Erfindung sieht vor, dass eine Eingabeeinrichtung vorgesehen ist, mit der ein Benutzer den Rechner veranlassen kann, in einem ausgewählten Bereich der dargestellten Messergebnisse der optischen Kohärenztomographie einen zusätzlichen Abtrag von Korneagewebe mit dem ersten Laser zu bewirken oder in einem ausgewählten Bereich den Abtrag von Korneagewebe zu reduzieren.

Die Erfindung lehrt auch den Einsatz extrem schneller Einrichtungen für die OCT unter Einsatz von Femtosekunden-Strahlungsquellen, bevorzugt mit Repetitionsraten im Bereich von 10 GHz und bevorzugt im Bereich von 100 GHz oder mehr, insbesondere den Einsatz sogenannter VECSELs oder VCSELs (Vertical External Cavity Surface Emitting Laser). Solche Halbleiter-Laserdioden können elektrisch oder optisch gepumpt werden und erreichen trotz einer Baugröße im Zentimeterbereich sehr hohe Leistungen und Wirkungsgrade. Auch können Femtosekunden-Faserlaser für die Erfindung eingesetzt werden. Die Erfindung lehrt auch den Einsatz solcher Strahlungsquellen mit Erzeugung von Fs-Superkontinua mit Bandbreiten größer 100 nm bis zu 1000 nm und mit Repititionsraten größer 100 GHz, so dass eine extrem hohe Messrate erreichbar ist, also die Erzeugung von Abbildungen von Strukturen der Kornea auf zum Beispiel einem Bildschirm mit geringster Zeitverzögerung gegenüber dem bei einem Ablationsvorgang tatsächlich momentan erzielten Zustand der Kornea, d.h. der tatsächliche Zustand der Kornea wird quasi in Echtzeit (ohne Zeitverzögerung) bildlich dargestellt und kann auch ohne Zeitverzögerung rechnerisch bearbeitet werden.

Weitere bevorzugte Ausgestaltungen des erfindungsgemäßen Systems für die refraktive ophthalmologische Chirurgie sind in den abhängigen Ansprüchen beschrieben.

Nachfolgend wird ein Ausführungsbeispiel der Erfindung anhand der Zeichnungen näher beschrieben. Es zeigt:
Figur 1 schematisch ein System für die refraktive ophthalmologische Chirurgie; und
Figur 2 schematisch eine Anzeigeeinrichtung zum Anzeigen von mit optischer Kohärenztomographie gewonnenen Daten.

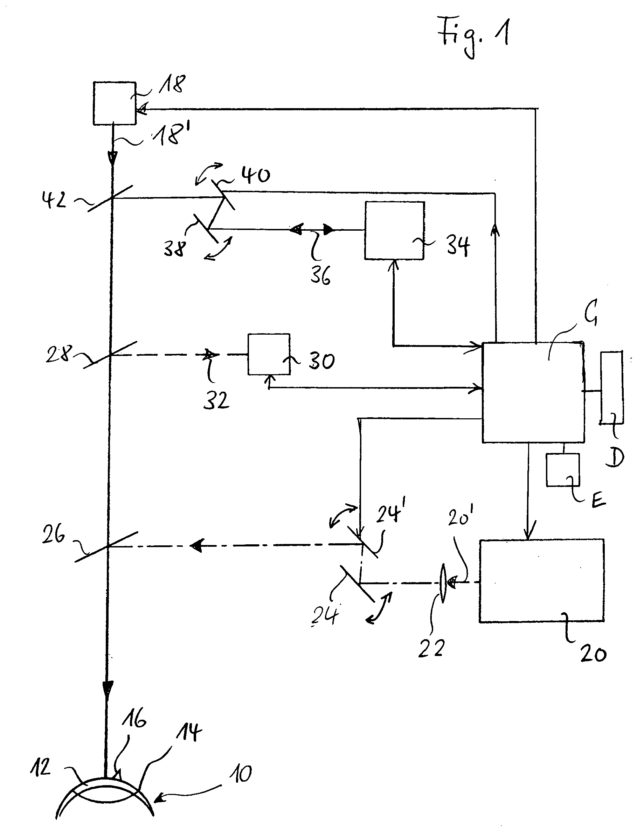

Figur 1 zeigt ein mit refraktiver ophthalmologischer Chirurgie zu behandelndes Auge 10, schematisch dessen Kornea 12, den Pupillenrand 14 und eine sogenannte Irregularität 16 der Hornhaut, hier in Form eines sogenannten "central island", also eines Vorsprunges auf der Kornea mit Abmessungen im mm- bis µm-Bereich.

Das System weist in bekannter Weise einen Fixationslaser 18 auf, der einen (schwachen) Laserstrahl 18' emittiert und vom Patienten zur Fixierung des Auges anvisiert wird.

In an sich bekannter Weise weist das System wie bei einer LASIK-Vorrichtung einen UV-Laser 20 auf, zum Beispiel einen Excimer-Laser, der Strahlung 20' mit einer Wellenlänge von 193 nm emittiert, die über eine Linse 22 auf Scanner-Spiegel 24, 24' gerichtet und über einen Umlenkspiegel 26 auf das Auge 10 gelenkt wird. Ein Rechner C steuert den Laser 20 und die Scanner-Spiegel 24, 24' gemäß einem zuvor berechneten Ablationsprofil in an sich herkömmlicher Weise. Mit dem dargestellten System wird in bekannter Weise zum Beispiel eine LASIK durchgeführt.

Das System weist weiterhin einen sogenannten Eye-Tracker auf. Der Eye-Tracker enthält eine Kamera 30, mit der vom Auge 10 Bilder über einen Umlenkspiegel 28 in Richtung des Pfeiles 32 aufgenommen werden, die dann in an sich bekannter Weise einer Bildverarbeitung unterzogen werden, um Bewegungen des Auges, die der Patient häufig trotz des Fixationslasers 18 nicht vermeiden kann, zu verfolgen und die Steuerung der Scannner-Spiegel 24, 24' für den Laserstrahl 20' den Augenbewegungen entsprechend nachzuführen, sodass das Ablationsprofil möglichst ortstreu abgetragen wird.

Die digital gewonnenen Aufnahmen der Kamera 30 werden im Rechner C verarbeitet und entsprechend steuert der Rechner C dann die Scanner-Spiegel 24, 24' bezüglich des Ablationsstrahls 20'.

In das System für die LASIK integriert ist eine Einrichtung 34 für die optische Kohärenztomographie, die einen entsprechenden Laser in an sich bekannter Weise enthält. Die Einrichtung 34 für die optische Kohärenztomographie emittiert Strahlung und empfängt Strahlung gemäß dem Doppelpfeil 36. Die Abtastung erfolgt über Scanner-Spiegel 38, 40. Die Wechselwirkung des Rechners C mit den einzelnen Komponenten ist in Figur 1 mit Leitungen und Pfeilen angedeutet. Entsprechend steuert der Rechner C die Einrichtung 34 für die optische Kohärenztomographie und die hierzu gehörenden Scanner-Spiegel 38, 40.

Die Einrichtung 34 für die optische Kohärenztomographie arbeitet mit Abtastraten im Bereich von wenigen MHz bis zu Abtastraten im GHz-Bereich, in Abhängigkeit von der verwendeten Strahlungsquelle, so dass Messzeiten für eine gesamte zu vermessende Fläche (also entsprechend etwa dem Ablationsbereich der Kornea) erreicht werden, die deutlich kleiner als 1 Sekunde sind.

Die Strahlungsquelle der Einrichtung 34 für die optische Kohärenztomographie, z. B. ein Laser, ist eine extrem breitbandige Strahlungsquelle mit einer Breitbandigkeit deutlich größer als 100 nm und mit sehr hohen Repetitionsraten größer als 10 MHz bis mehr als 100 GHz. Dies ermöglicht eine hohe dreidimensionale Auflösung im Bereich von 10 µm und besser. Dabei kann ein interessierendes Abbild einer Fläche der Kornea, zum Beispiel die momentane Oberfläche während der sukzessiven Ablation, in weniger als 1 Sekunde vermessen und über den Rechner C auf einer Anzeigeeinrichtung D dargestellt werden. Die breitbandige Strahlung mit Δλ >> 100 nm hat Mittenwellenlängen λ im Bereich von etwa 800 bis 1300 nm. Damit wird gemäß dem gezeigten Ausführungsbeispiel die Topographie der Kornea, deren Dicke, die Ausdehnung der Augenvorderkammer und die lokale Lage der angrenzenden Strukturen, wie Iris und Augenlinse, vermessen und entsprechende geometrische Abbilder dieser Strukturen werden über den Rechner C auf der Anzeigeeinrichtung D wahlweise und in gewünschter Kombination dargestellt.

Die Tiefenauflösung (üblicherweise als z-Richtung bezeichnet) liegt dabei auch im µm-Bereich, zum Beispiel besser als 3 µm, während in Querrichtung (üblicherweise als xy-Richtung bezeichnet) ebenfalls Auflösungen deutlich besser als 10 µm erreicht werden. Damit können Substrukturen der Kornea, wie das Epithel, die Bowman-Membran oder die Lage des Mikrokeratom-Schnittes bei der LASIK gut erkannt werden.

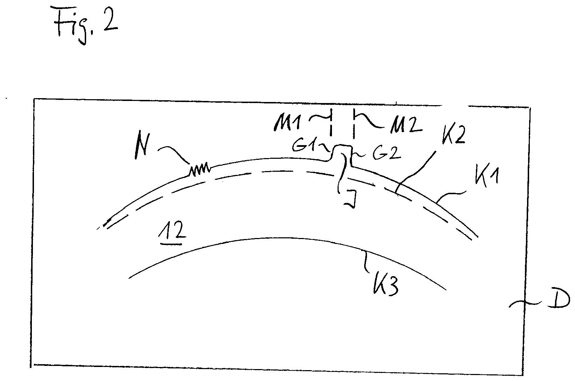

Figur 2 zeigt schematisch eine Darstellung mit OCT-vermessenen Strukturen auf der Anzeigeeinrichtung D gemäß einer Steuerung durch den Rechner C. Gezeigt ist die vor Beginn der Ablation gegebene Oberfläche K1 der Kornea mit einer ersten Irregularität I in Form eines "central island", also eines Vorsprunges. Der Vorsprung hat Abmessungen im mm- bis µm-Bereich (ist also in der Figur extrem gegenüber den Abmessungen der Kornea vergrößert). Die beiden Flanken des Vorsprunges haben Gradienten G1, G2, d.h. Steigungen gegenüber der angrenzenden Fläche K1 der Kornea 12. Weiterhin hat beim dargestellten Beispiel die Oberfläche K1 der Kornea 12 Narben N, die ebenfalls Abmessungen im mm- bis µm-Bereich haben.

Weiterhin zeigt Figur 2 die aufgrund des berechneten Abtrags des Ablationsprofils anzustrebenden Soll-Oberfläche K2 der Kornea, also das anzustrebende Operationsergebnis. Figur 2 zeigt auch die innere Oberfläche K3 der Kornea schematisch.

Mit der Einrichtung 34 für die optische Kohärenztomographie und mit dem deren Messergebnisse verarbeitenden Rechner C kann die Kurve K1 gemäß Figur 2 erzeugt und auf dem Anzeigegerät D dargestellt werden. Dabei ist der Rechner C so programmiert, dass er die genannten Irregularitäten mit Abmessungen im µm-Bereich erkennt und gemäß einer Option des Benutzers hervorgehoben, zum Beispiel farbig oder durch starke Linien, darstellt. Mit an sich bekannten Verfahren der Bildverarbeitung kann der Rechner aufgrund der Gradienten G1, G2 die Irregularitäten durch Vergleich mit vorgebbaren Schwellenwerten "erkennen" und darstellen. Zum Beispiel kann bei der Bildverarbeitung durch Vergleich von nebeneinander liegenden Messpunkt-Bereichen ein Gradient G1, G2 erkannt werden, der über einem vorgegebenen Schwellenwert liegt und anzeigt, dass an dieser Stelle möglicherweise eine Irregularität vorliegt. Dem Benutzer kann dann gemäß einer bevorzugten Ausgestaltung die Option an die Hand gegeben werden, in diesem kritischen Bereich eine vergrößerte Darstellung des momentanen Zustandes der Kornea aufzurufen und darzustellen. Diese Darstellung kann während der Durchführung der Ablation, also dem schrittweisen Abtrag von Korneagewebe Schicht-für-Schicht, dauernd "on-line" wiederholt werden, sodass der Arzt den Behandlungsfortschritt, d.h. den zeitlichen Vorgang des sukzessiven Abtrags von Korneagewebe Schicht-für-Schicht aufgrund der kohärenztomographischen Messungen auf dem Anzeigegerät D verfolgen kann. Erkennt der Arzt dabei aufgrund der Irregularitäten gewisse Problembereiche, in denen eine stärkere Ablation oder auch eine schwächere Ablation als ursprünglich gemäß dem Ablationsprofil vorgesehen, erforderlich sind, dann kann er direkt gemäß einer bevorzugten Ausgestaltung in den Prozess eingreifen. Erkennt der Arzt zum Beispiel auf der Darstellung eine Irregularität entsprechend dem Vorsprung I ("central island"), dann kann er Markierer M1, M2 setzen, zwischen denen in diesem Fall ein erhöhter Abtrag von Korneagewebe, im Vergleich zu den umgebenden Bereichen der Kornea, erforderlich ist und er kann über eine Eingabeeinrichtung E die Markierer M1, M2 entsprechend zur Eingrenzung dieses Bereiches setzen. Es versteht sich, dass diese Markierer 3-dimensional anzusetzen sind, also auch in Tiefe der Zeichnungsebene etwa die Abmessung haben, die in der Darstellungsebene gegeben ist. Der Arzt kann dann über die Eingabeeinrichtung E vorgeben, wie stark der zusätzliche Gewebeabtrag in dem Bereich der Irregularität sein soll.

Entsprechendes gilt für den Bereich der Narben N, in dem dann die Ablation gegebenenfalls verringert werden muss, um im Ergebnis in Fällen aller Irregularitäten dann eine relativ glatte Korneaoberfläche zu gewinnen, wie sie in Figur 2 mit K2 bezeichnet ist.

### Bezugszeichenliste

- 10: Auge
- 12: Kornea
- 14: Pupillenrand
- 16: Narbe oder "central island"
- 18: Fixationslaser
- 18': Fixationslaserstrahl
- 20: UV-Laser
- 22: Linse
- 24: Scanner-Spiegel
- 24': Scanner-Spiegel
- 26: Umlenkspiegel
- 28: Umlenkspiegel
- 30: Kamera
- 32: Pfeil
- 34: OCT-System
- 36: Doppel-Pfeil
- 38: Scanner-Spiegel
- 40: Scanner-Spiegel
- C: Rechner
- D: Display
- E: Eingabe
- I: Central Island
- N: Narbe(n)
- M1, M2: Marker
- G1, G2: Gradienten
- K1, K2, K3, K4: Flächen

## Patentansprüche

1. System für die refraktive ophthalmologische Chirurgie mit
a) einem ersten Laser (20) zum Abtrag von Korneagewebe,
b) Mitteln (22, 24, 24') zum zeit- und ortsgesteuerten Führen der Strahlung (20') des ersten Lasers (20) auf das Auge (10),
c) einer Einrichtung (34) für eine optische Kohärenztomographie mit einem zweiten Laser zur Durchführung von optischer Kohärenztomographie am Auge (10),
d) Mitteln (38, 40) zum zeit- und ortsgesteuerten Führen der Strahlung des zweiten Lasers auf das Auge (10), und mit
e) einem Rechner (C), der
e1) bei dem Abtrag von Korneagewebe gemäß einem Programm den ersten Laser (20) und die genannten Mittel (22, 24, 24') zum zeit- und ortsgesteuerten Führen der Strahlung (20') des ersten Lasers (20) auf das Auge zur Erzielung einer Neuformung der Kornea (12) steuert,
e2) die Einrichtung (34) für die optische Kohärenztomographie steuert und so programmiert ist, dass während des Abtrags, und vor und nach dem Abtrag von Korneagewebe Messungen an der Kornea (12) durchgeführt werden,
e4) unter vorgebbaren Bedingungen den Programmablauf für den Abtrag von Korneagewebe im Anschluss an eine Messung mittels der optischen Kohärenztomographie in Abhängigkeit von dem Ergebnis der Messung steuert,
wobei der Rechner
e3) programmiert ist, um bei der optischen Kohärenztomographie gewonnene Messergebnisse während des Abtrags von Korneagewebe auf einer Anzeigeeinrichtung (D) darzustellen,
**dadurch gekennzeichnet, dass** der Rechner
e3') programmiert ist, um bei der optischen Kohärenztomographie gewonnene Messergebnisse auch vor dem Abtrag von Korneagewebe auf einer Anzeigeeinrichtung (D) darzustellen,
und dass eine Eingabeeinrichtung (E) vorgesehen ist, mit der ein Benutzer den Rechner (C) veranlassen kann, in einem ausgewählten Bereich (M1, M2) der dargestellten Messergebnisse der optischen Kohärenztomographie einen zusätzlichen Abtrag von Korneagewebe mit dem ersten Laser (20) zu bewirken oder in einem ausgewählten Bereich (N) den Abtrag von Korneagewebe zu reduzieren;
wobei der Rechner (C) so programmiert ist, dass die mit der optischen Kohärenztomographie gewonnenen Messergebnisse darauf analysiert werden, ob eine zu ablatierende Korneaoberfläche eine Irregularität (I, N) aufweist, wobei die genannte Analyse die Ermittlung von Gradienten der Korneaoberfläche beinhaltet.

2. System für die refraktive ophthalmologische Chirurgie gemäß Anspruch 1, **dadurch gekennzeichnet, dass** die Darstellung der mit der optischen Kohärenztomographie gewonnenen Messergebnisse ein zumindest 2-dimensionales Abbild (K1, K2, K3) einer Kurve beinhaltet, die vor und/oder während des Abtrags von Korneagewebe den jeweils aktuellen Zustand einer zu bearbeitenden oder bearbeiteten Korneaoberfläche wiedergibt.

3. System für die refraktive ophthalmologische Chirurgie gemäß den Ansprüchen 1 und 2, **dadurch gekennzeichnet, dass** der Rechner (C) so programmiert ist, dass durch den Benutzer der ausgewählte Bereich (M1, M2) in Bezug auf das Abbild (K1, K2, K3) markierbar ist.

## Claims

1. System for refractive ophthalmic surgery, comprising
a) a first laser (20) for ablating corneal tissue,
b) means (22, 24, 24') for temporally and spatially controlled guidance of the radiation (20') of the first laser (20) onto the eye (10),
c) a device (34) for optical coherence tomography, comprising a second laser for carrying out optical coherence tomography on the eye (10),
d) means (38, 40) for temporally and spatially controlled guidance of the radiation of the second laser onto the eye (10), and comprising
e) a computer (C), which
e1) controls the first laser (20) and the aforementioned means (22, 24, 24') for temporally and spatially controlled guidance of the radiation (20') of the first laser (20) onto the eye during the ablation of corneal tissue in accordance with a program for the purposes of obtaining a reformation of the cornea (12),
e2) controls the device (34) for optical coherence tomography and is programmed in such a way that during the ablation, and before and after the ablation of corneal tissue, measurements are performed on the cornea (12),
e4) controls the program sequence for ablating corneal tissue under predeterminable conditions following a measurement by means of optical coherence tomography, depending on the result of the measurement,
wherein the computer
e3) is programmed to display measurement results obtained during optical coherence tomography on a display device (D) during the ablation of corneal tissue,
**characterized in that** the computer
e3') is programmed to display measurement results obtained during optical coherence tomography on a display device (D) even before the ablation of corneal tissue,
and **in that** provision is made of an input device (E), by means of which a user is able to prompt the computer (C) to effect an additional ablation of corneal tissue with the first laser (20) in a selected region (M1, M2) of the depicted measurement results of optical coherence tomography or to reduce the ablation of corneal tissue in a selected region (N);
wherein the computer (C) is programmed in such a way that the measurement results obtained with optical coherence tomography are analysed as to whether a corneal surface to be ablated has an irregularity (I, N), wherein the aforementioned analysis contains ascertaining gradients of the corneal surface.

2. System for refractive ophthalmic surgery according to Claim 1, **characterized in that** the display of the measurement results obtained by optical coherence tomography contains an at least two-dimensional image (K1, K2, K3) of a curve which in each case reproduces the current state of a corneal surface to be worked on, or which has been worked on, during the ablation of corneal tissue.

3. System for refractive ophthalmic surgery according to Claims 1 and 2, **characterized in that** the computer (C) is programmed in such a way that the user is able to mark the selected region (M1, M2) in relation to the image (K1, K2, K3).

## Revendications

1. Système pour la chirurgie ophtalmologique réfractive, comprenant
a) un premier laser (20) destiné à enlever du tissu cornéen,
b) des moyens (22, 24, 24') destinés au guidage commandé en temps et en lieu du rayonnement (20') du premier laser (20) sur l'oeil (10),
c) un dispositif (34) pour une tomographie en cohérence optique comprenant un deuxième laser destiné à effectuer une tomographie en cohérence optique sur l'oeil (10),
d) des moyens (38, 40) destinés au guidage commandé en temps et en lieu du rayonnement du deuxième laser sur l'oeil (10), et comprenant
e) un ordinateur (C) qui
e1) lors de l'enlèvement du tissu cornéen, commande le premier laser (20) et lesdits moyens (22, 24, 24') destinés au guidage commandé en temps et en lieu du rayonnement (20') du premier laser (20) sur l'oeil conformément à un programme en vue d'obtenir un refaçonnage de la cornée (12),
e2) commande le dispositif (34) pour la tomographie en cohérence optique et est programmé de telle sorte que des mesures sont effectuées sur la cornée (12) pendant l'enlèvement et aussi avant et après l'enlèvement de tissu cornéen,
e4) sous des conditions pouvant être prédéfinies, commande le déroulement du programme pour l'enlèvement de tissu cornéen à la suite d'une mesure au moyen de la tomographie en cohérence optique en fonction du résultat de la mesure,
l'ordinateur
e3) étant programmé pour représenter sur un dispositif d'affichage (D) pendant l'enlèvement du tissu cornéen des résultats de mesure obtenus lors de la tomographie en cohérence optique,
**caractérisé en ce que** l'ordinateur
e3') est programmé pour représenter sur un dispositif d'affichage (D) des résultats de mesure obtenus lors de la tomographie en cohérence optique également avant l'enlèvement du tissu cornéen,
et **en ce qu'**il existe un dispositif de saisie (E) avec lequel un utilisateur peut amener l'ordinateur (C) à provoquer un enlèvement supplémentaire de tissu cornéen avec le premier laser (20) dans une zone (M1, M2) sélectionnée des résultats de mesure représentés de la tomographie en cohérence optique, ou à réduire l'enlèvement de tissu cornéen dans une zone (N) sélectionnée ;
l'ordinateur (C) étant programmé de telle sorte que les résultats de mesure obtenus avec la tomographie en cohérence optique sont soumis à une analyse afin de déterminer si une surface cornéenne où il faut pratiquer une ablation présente une irrégularité (I, N), ladite analyse incluant la détermination de gradients de la surface cornéenne.

2. Système pour la chirurgie ophtalmologique réfractive selon la revendication 1, **caractérisé en ce que** la représentation des résultats de mesure obtenus avec la tomographie en cohérence optique contient une représentation (K1, K2, K3) au moins bidimensionnelle d'une courbe qui reproduit avant et/ou pendant l'enlèvement du tissu cornéen l'état respectif actuel d'une surface cornéenne à traiter ou traitée.

3. Système pour la chirurgie ophtalmologique réfractive selon les revendications 1 et 2, **caractérisé en ce que** l'ordinateur (C) est programmé de telle sorte que la zone (M1, M2) sélectionnée peut être marquée par l'utilisateur en référence à la représentation (K1, K2, K3) .
